# EUROPEAN PATENT APPLICATION

(11) **EP 3 326 658 A1**
(43) Date of publication of application: **30.05.2018**
(21) Application number: 17204136.0
(22) Date of filing: 28.11.2017
(51) Int. Cl.: A61L 2/20, A61L 2/16, A61L 2/18, A61L 2/24, A61L 2/28

(54) **STERILIZATION SYSTEM WITH INDEPENDENT VACUUM CHAMBERS**

(30) Priority: 29.11.2016 US 201662427255 P; 09.06.2017 US 201715618295
(71) Applicant: Ethicon, Incorporated, Somerville, NJ 08876 (US)
(72) Inventor: OMIDBAKHSH, Navid, Irvine, CA California 92618 (US)
(74) Representative: Carpmaels & Ransford LLP

(57) **Abstract**

A sterilization system includes a first chamber, a second chamber, a sterilization module, and a pump assembly. The first chamber includes a first venting valve. The first chamber is configured to receive a first medical device. The first chamber is further operable to sterilize the first medical device. The first sterilization module is operable to dispense sterilant into the first chamber. The pump assembly is in fluid communication with the first chamber and the second chamber. The pump assembly is operable to selectively and repeatedly transfer at least some of the sterilant from the first chamber to the second chamber. The pump assembly is further operable to selectively and repeatedly transfer at least some of the sterilant from the second chamber to the first chamber.

## Description

### BACKGROUND

Re-usable medical devices such as certain surgical instruments, endoscopes, etc., may be sterilized before re-use in order to minimize the likelihood that a contaminated device might be used on a patient, which could cause an infection in the patient. Various sterilization techniques may be employed, such as steam, hydrogen peroxide, peracetic acid, and vapor phase sterilization, either with or without a gas plasma and ethylene oxide (EtO). Each of these methods may depend to a certain extent on the diffusion rates of the sterilization fluids (e.g., gases) upon or into the medical devices to be sterilized.

Before sterilization, medical devices may be packaged within containers or pouches having a semi-permeable barrier that allows transmission of the sterilizing fluid-sometimes referred to as a sterilant-but prevents admission of contaminating organisms, particularly post-sterilization and until the package is opened by medical personnel. For the sterilization cycle to be efficacious, the contaminating organisms within the package must be killed because any organisms that survive the sterilization cycle could multiply and re-contaminate the medical device. Diffusion of the sterilant may be particularly problematic for medical devices that have diffusion-restricted spaces therein because these diffusion-restricted spaces may reduce the likelihood that a sterilization cycle may be effective. For example, some endoscopes have one or more long narrow lumens into which the sterilant must diffuse in sufficient concentration for sufficient time to achieve a successful sterilization cycle.

Sterilization of medical devices may be performed with an automated sterilization system such as a STERRAD® System by Advanced Sterilization Products of Irvine, California. Examples of automated sterilization systems are described in U.S. Pat. No. 6,939,519, entitled "Power System for Sterilization Systems Employing Low Frequency Plasma," issued September 6, 2005, the disclosure of which is incorporated by reference herein; U.S. Pat. No. 6,852,279, entitled "Sterilization with Temperature-Controlled Diffusion Path," issued February 8, 2005, the disclosure of which is incorporated by reference herein; U.S. Pat. No. 6,852,277, entitled "Sterilization System Employing a Switching Module Adapter to Pulsate the Low Frequency Power Applied to a Plasma," issued February 8, 2005, the disclosure of which is incorporated by reference herein; U.S. Pat. No. 6,447,719, entitled "Power System for Sterilization Systems Employing Low Frequency Plasma," issued September 10, 2002, the disclosure of which is incorporated by reference herein; and U.S. Provisional Pat. App. No. 62/316,722, entitled "System and Method for Sterilizing Medical Devices," filed April 1, 2016, the disclosure of which is incorporated by reference herein.

Some sterilization systems may use vaporized chemical sterilants or chemical gas such as hydrogen peroxide, peracetic acid, ozone, chlorine dioxide, nitrogen dioxide, etc., to sterilize medical devices. Examples of such systems are described in U.S. Pat. No. 6,365,102, entitled "Method of Enhanced Sterilization with Improved Material Compatibility," issued April 2, 2002, the disclosure of which is incorporated by reference herein, and U.S. Pat. No. 6,325,972, entitled "Apparatus and Process for Concentrating a Liquid Sterilant and Sterilizing Articles Therewith," issued December 4, 2001, the disclosure of which is incorporated by reference herein. Some such systems provide a hydrogen peroxide/gas plasma sterilization system comprising a vacuum chamber and plasma source and increased concentration of hydrogen peroxide for sterilization. Some such systems may have difficulty sterilizing lumens of some medical devices if their length exceeds a certain value; or the processing time of such systems may still not be fast enough for some applications. Thus, some medical devices such as long and/or narrow flexible endoscopes may not be completely sterilized by these systems due to the insufficient reach of sterilant vapor to the inside of the channels. Such medical devices might therefore only be disinfected without being sterilized. Sterilization systems that use ethylene oxide may have a relatively long processing time (e.g., longer than 24 hours), which may be undesirable in some cases.

Operator error may result in medical devices that are erroneously believed to be decontaminated being returned to service. Confirming that a sterilization cycle has been efficacious may help medical personnel avoid using a contaminated medical device on a patient. The sterilized medical device might not itself be checked for contaminating organisms because such an activity may introduce other contaminating organisms to the medical device, thereby re-contaminating it. Thus, an indirect check may be performed using a sterilization indicator. A sterilization indicator is a device that may be placed alongside or in proximity to a medical device being subject to a sterilization cycle, such that the sterilization indicator is subject to the same sterilization cycle as the medical device. For instance, a biological indictor having a predetermined quantity of microorganisms may be placed into a sterilization chamber alongside a medical device and subject to a sterilization cycle. After the cycle is complete, the microorganisms in the biological indicator may be cultured to determine whether any of the microorganisms survived the cycle. The presence or absence of living microorganisms in the biological indicator will indicate whether the sterilization cycle was effective.

While a variety of systems and methods have been made and used for surgical instrument sterilization, it is believed that no one prior to the inventor(s) has made or used the technology as described herein.

### BRIEF DESCRIPTION OF THE DRAWINGS

It is believed the present invention will be better understood from the following description of certain examples taken in conjunction with the accompanying drawings, in which like reference numerals identify the same elements and in which:
FIG. 1 depicts a schematic view of an exemplary medical device sterilizing cabinet;
FIG. 2 depicts a high level flowchart of an exemplary set of steps that the sterilizing cabinet of FIG. 1 could perform to sterilize a medical device;
FIG. 3 depicts a flowchart of an exemplary set of steps that may be carried out as part of a sterilization cycle within the set of steps of FIG. 2;
FIG. 4 depicts a schematic view of another exemplary medical device sterilizing cabinet;
FIG. 5 depicts a high level flowchart of an exemplary set of steps that the sterilizing cabinet of FIG. 4 could perform to sterilize a medical device;
FIG. 6 depicts a flowchart of an exemplary set of steps that may be carried out as part of a sterilization cycle within the set of steps of FIG. 5;
FIG. 7 depicts a flowchart of an exemplary set of steps that may be carried out to apply a vacuum to sterilization chambers of the sterilizing cabinet of FIG. 4, as part of the sterilization cycle of FIG. 6; and
FIG. 8 depicts a flowchart of an exemplary alternative set of steps that may be carried out to apply a vacuum to sterilization chambers of the sterilizing cabinet of FIG. 4, as part of the sterilization cycle of FIG. 6.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

### I. Overview of Exemplary Sterilization System

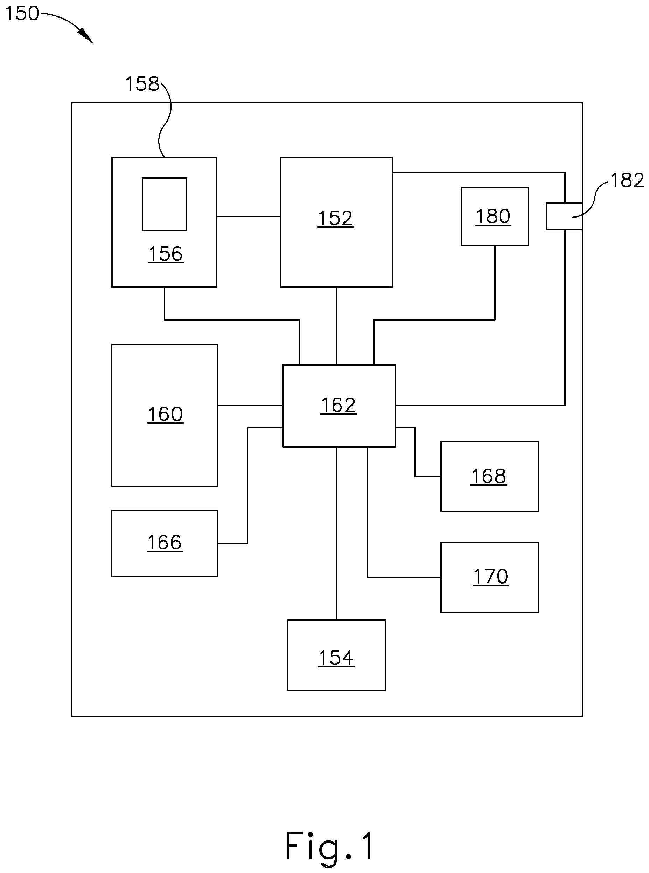

FIG. 1 depicts an exemplary sterilizing cabinet (150) that is operable to sterilize medical devices such as endoscopes, etc. Sterilizing cabinet (150) of the present example includes a sterilization chamber (152), which is configured to receive one or more medical devices for sterilization. In some other versions (e.g., as described further below), sterilizing cabinet (150) may include more than one sterilization chamber (152). While not shown, sterilizing cabinet (150) also includes a door that opens and closes sterilization chamber (152) in response to actuation of a kick plate. An operator may thereby open and close sterilization chamber (152) in a hands-free fashion. Of course, any other suitable features may be used to provide selective access to sterilization chamber. Sterilizing cabinet (150) also includes a sterilization module (156) that is operable to dispense a sterilant into sterilization chamber (152) in order to sterilize medical devices contained in sterilization chamber (152). In the present example, sterilization module (156) is configured to receive replaceable sterilant cartridges (158) containing a certain amount of sterilant. By way of example only, each sterilant cartridge (158) may contain enough sterilant to perform five sterilization procedures.

In the present example, sterilization module (156) is operable to apply a sterilant in the form of a vapor within sterilization chamber (152). By way of example only, sterilization module (156) may comprise a combination of a vaporizer and a condenser. The vaporizer may include a chamber that receives a particular concentration of sterilant solution (e.g., a liquid hydrogen peroxide solution with a concentration of about 59% nominal, or between about 20% and about 59%, or between about 53% and about 59.6%); where the sterilant solution changes phase from liquid to vapor. The condenser may provide condensation of the sterilant solution vapor, and the concentration of the sterilant solution may be thereby increased (e.g., from about 59% nominal to somewhere between about 83% nominal and about 95% nominal), by removal of water vapor. Alternatively, any other suitable methods and components may be used to apply sterilant in the form of a vapor within sterilization chamber (152). It should also be understood that condensation within sterilization chamber (152) may serve as a potential reservoir of sterilant that could be tapped by manipulation of conditions in a sterilization chamber (152) to re-vaporize the condensation.

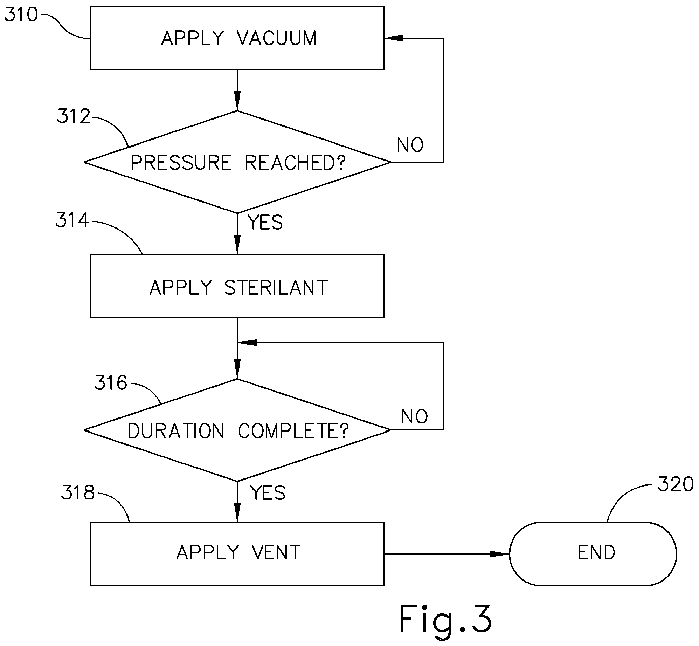

In some examples, to supplement the application of the sterilant in the form of a vapor, the sterilant may also be applied to the inside of lumen(s) and/or other internal spaces within the medical device and/or the outside of the medical device, before the medical device is placed in sterilization chamber (152). By way of example only, sterilant may be applied in liquid form to the inside of lumen(s) and/or other internal spaces within the medical device and/or the outside of the medical device. As another merely illustrative example, a capsule that contains liquid sterilant may be placed in in fluid communication with the lumen(s) after activation of sterilization cabinet (150). In versions where a sterilant is applied to the inside of lumen(s) and/or other internal spaces within the medical device and/or the outside of the medical device, before the medical device is placed in sterilization chamber (152), the sterilant may evaporate while a vacuum is applied to sterilization chamber (152) (e.g., as described in greater detail below with reference to block 310 of FIG. 3) and even after vacuum is applied; and provide more concentration of sterilant to the areas of the medical device with less penetration range, thereby further promoting effective sterilization.

Sterilizing cabinet (150) of the present example further includes a touch screen display (160). Touch screen display (160) is operable to render the various user interface display screens, such as those described in U.S. Provisional Pat. App. No. 62/316,722, the disclosure of which is incorporated by reference herein. Of course, touch screen display (160) may display various other screens as well. Touch screen display (160) is further configured to receive user input in the form of the user contacting touch screen display (160) in accordance with conventional touch screen technology. In addition, or in the alternative, sterilizing cabinet (150) may include various other kinds of user input features, including but not limited to buttons, keypads, keyboards, a mouse, a trackball, etc.

Sterilizing cabinet (150) of the present example further includes a processor (162), which is in communication with sterilization module (156) and with touch screen display (160). Processor (162) is operable to execute control algorithms to drive sterilization module (156) in accordance with user input. Processor (162) is further operable to execute instructions to display the various screens on touch screen display (160); and to process instructions received from a user via touch screen display (160) (and/or via other user input features). Processor (162) is also in communication with various other components of sterilization cabinet (150) and is thereby operable to drive those components and/or process input and/or other data from those components. Various suitable components and configurations that may be used to form processor (162) will be apparent to those of ordinary skill in the art in view of the teachings herein.

Sterilizing cabinet (150) of the present example further includes an identification tag reader (166), which is operable to read an identification tag of a biological indicator as described herein. By way of example only, identification tag reader (166) may comprise an optical reader that is operable to read an optical identification tag (e.g., barcode, QR code, etc.) of a biological indicator. In addition, or in the alternative, identification tag reader (166) may comprise RFID reader that is operable to read an RFID identification tag of a biological indicator. Various suitable components and configurations that may be used to form identification tag reader (166) will be apparent to those of ordinary skill in the art in view of the teachings herein. Data received through identification tag reader (166) is processed through processor (162). Such data may indicate the contents of the biological indicator, the source of the biological indicator, other identifying information associated with the biological indicator, and/or various other kinds of information as will be apparent to those of ordinary skill in the art.

Sterilizing cabinet (150) of the present example further includes a memory (168), which is operable to store control logic and instructions and that are executed by processor (162) to drive components such as sterilization module (156), touch screen display (160), communication module (154), and identification tag reader (166). Memory (168) may also be used to store results associated with setup of a sterilization cycle, performance of a load conditioning cycle, performance of a sterilization cycle, and/or various other kinds of information. Various suitable forms that memory (168) may take, as well as various ways in which memory (168) may be used, will be apparent to those of ordinary skill in the art in view of the teachings herein.

Sterilizing cabinet (150) of the present example further includes a printer (170), which is operable to print information such as results associated with setup of a sterilization cycle, performance of a load conditioning cycle, performance of a sterilization cycle, and/or various other kinds of information. By way of example only, printer (170) may comprise a thermal printer, though of course any other suitable kind of printer may be used. Various suitable forms that printer (170) may take, as well as various ways in which printer (170) may be used, will be apparent to those of ordinary skill in the art in view of the teachings herein. It should also be understood that printer (170) is merely optional and may be omitted in some versions.

Sterilizing cabinet (150) of the present example further includes a vacuum source (180) and a venting valve (182). Vacuum source (180) is in fluid communication with sterilization chamber (152) and is also in communication with processor (162). Thus, processor (162) is operable to selectively activate vacuum source (180) in accordance with one or more control algorithms. When vacuum source (180) is activated, vacuum source (180) is operable to reduce the pressure within sterilization chamber (152) as will be described in greater detail below. Venting valve (182) is also in fluid communication with sterilization chamber (152). In addition, venting valve (182) is in communication with processor (162) such that processor (162) is operable to selectively activate venting valve (182) in accordance with one or more control algorithms. When venting valve (182) is activated, venting valve (182) is operable to vent sterilization chamber (152) to atmosphere as will be described in greater detail below. Various suitable components that may be used to provide vacuum source (180) and venting valve (182) will be apparent to those of ordinary skill in the art in view of the teachings herein.

In addition to the foregoing, sterilizing cabinet (150) may be configured and operable in accordance with at least some of the teachings of U.S. Pat. No. 6,939,519, the disclosure of which is incorporated by reference herein; U.S. Pat. No. 6,852,279, the disclosure of which is incorporated by reference herein; U.S. Pat. No. 6,852,277, the disclosure of which is incorporated by reference herein; U.S. Pat. No. 6,447,719, the disclosure of which is incorporated by reference herein; U.S. Pat. No. 6,365,102, the disclosure of which is incorporated by reference herein; U.S. Pat. No. 6,325,972, the disclosure of which is incorporated by reference herein; and/or U.S. Provisional Patent App. No. 62/316,722, the disclosure of which is incorporated by reference herein.

### II. Overview of Exemplary Sterilization Process

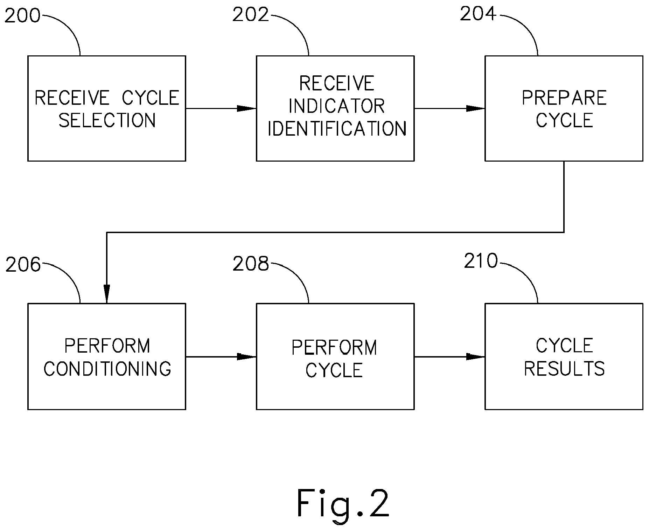

FIG. 2 depicts a high level flowchart of an exemplary set of steps that sterilizing cabinet (150) could perform to sterilize a used medical device, such as an endoscope. Sterilizing cabinet (150) may be configured to perform one or more sterilization cycles, with different sterilization cycles being appropriate for different types and quantities of medical devices. Thus, as an initial step, sterilizing cabinet (150) may display one or more available sterilization cycles via touch screen display (160) and then receive a sterilization cycle selection (block 200) from the user.

Sterilizing cabinet (150) may also display instructions indicating whether a biological indicator should be used with the selected sterilization cycle, and receive a biological indicator identification (block 202). Such a biological indicator identification (block 202) may be provided via identification tag reader (166), via touch screen display (160), or otherwise. A biological indicator may be placed inside sterilization chamber (152) of sterilizing cabinet (150) before the sterilization cycle begins and may remain in sterilization chamber (152) during the sterilization cycle. The user may thus identify the particular biological indicator (block 202) before the biological indicator is placed in sterilization chamber (152). In versions where more than one sterilization chamber (152) is included in a sterilizing cabinet (150), separate biological indicators may be placed in separate sterilization chambers (152). Each biological indicator may contain microorganisms that are responsive to a particular sterilization cycle. Upon completion of the sterilization cycle, the biological indicator may be tested for the microorganisms in order to provide a measure of the effectiveness of the sterilization cycle. A biological indicator may not necessarily be required for all sterilization cycles, but may be required based on hospital rules or local regulations.

Selection of a sterilization cycle (block 200) and identification of a biological indicator (block 202) may define one or more requirements for the configuration and arrangement of medical devices within sterilization chamber (152). Thus, in order to provide preparation for the sterilization cycle (204) once the sterilization cycle has been selected (block 200) and the biological indicator has been identified (block 202), sterilizing cabinet (150) may provide a display via touch screen display (160) indicating a proper medical device placement. This display may serve as a visual guide to a user's placement of medical device(s) (and perhaps a biological indicator) within sterilization chamber (152) of sterilizing cabinet (150), based on the sterilization cycle selection (block 200). A door of sterilization chamber (152) may be opened to enable the user to place the medical device(s) (and perhaps a biological indicator) within sterilization chamber (152) as instructed.

Once the user has placed the medical device in sterilization chamber (152) based on these instructions, the user may press a start button or other button indicating that medical device placement is complete. In some versions, sterilizing cabinet (150) is configured to automatically verify proper medical device placement. By way of example only, sterilizing cabinet (150) may employ photo sensors, imaging devices, weight sensors, and/or other components to verify proper medical device placement in sterilization chamber (152). It should be understood, however, that some versions of sterilizing cabinet (150) may lack the capability of automatically verifying proper placement of a medical device within sterilization chamber (152).

If medical device placement is verified and/or the user has otherwise completed the cycle preparation (block 204), sterilizing cabinet (150) may start a load conditioning process (block 206). Load conditioning process (block 206) prepares sterilization chamber (152) and the medical device(s) within sterilization chamber (152) for optimal sterilization during a sterilization cycle. Conditioning may include controlling and optimizing one or more characteristics of sterilization chamber (152). For example, during load conditioning, sterilizing cabinet (150) may continuously monitor the level of moisture within sterilization chamber (152) while reducing the level of moisture by, for example, circulating and dehumidifying the air of sterilization chamber (152), creating a vacuum within sterilization chamber (152), heating sterilization chamber (152), and/or other methods for dehumidifying a sealed chamber. This may continue until sterilizing cabinet (150) determines that an acceptable level of moisture has been reached.

As part of load conditioning cycle (block 206), sterilizing cabinet (150) may also continuously detect the temperature within sterilization chamber (152) while heating sterilization chamber (152) by, for example, convection of heated air, conduction through an interior surface of sterilization chamber (152), and/or using other techniques. This may continue until sterilizing cabinet (150) determines that an acceptable internal temperature has been reached. Various conditioning actions such as controlling temperature or humidity may be performed in parallel or in sequence. It should also be understood that load conditioning cycle (block 206) may verify that sterilization chamber (152) is sealed; verifying contents of sterilization chamber (152); checking physical characteristics of the contents of sterilization chamber (152) such as content volume, content weight, or other characteristics; and/or performing one or more conditioning steps that may include chemical treatment, plasma treatment, or other types of treatment to reduce moisture, raise temperature, and/or otherwise prepare the medical devices in sterilization chamber (152) for sterilization cycle (block 208).

While the one or more conditioning actions are being performed as part of load conditioning cycle (block 206), sterilizing cabinet (150) may display information via touch screen display (160) indicating to a user the duration of time before sterilization cycle (block 208) performance may begin. Once all load conditioning criteria have been successfully met, load conditioning cycle (block 206) is complete, and sterilization cycle (block 208) may then be performed. It should therefore be understood that sterilizing cabinet (150) is configured such that sterilization cycle (block 208) is not actually initiated until after load conditioning cycle (block 206) is complete. It should also be understood that load conditioning cycle (block 206) may be omitted or varied in some versions of sterilizing cabinet (150) operation.

As noted above, sterilization cabinet (150) may begin performing the sterilization cycle (block 208) automatically and immediately after load conditioning (block 206) has been completed. Sterilization cycle (block 208) may include exposing the medical device(s) in the sterilizing chamber to pressurized sterilant gas, further heat treatment, chemical treatment, plasma treatment, vacuum treatment, and/or other types of sterilization procedures. During performance of sterilization cycle (block 208), sterilization cabinet (150) may display information via touch screen display (160) such as a duration remaining for sterilization cycle (block 208), the current stage of sterilization cycle (block 208) (e.g. plasma, vacuum, injection, heat, chemical treatment), and/or other information.

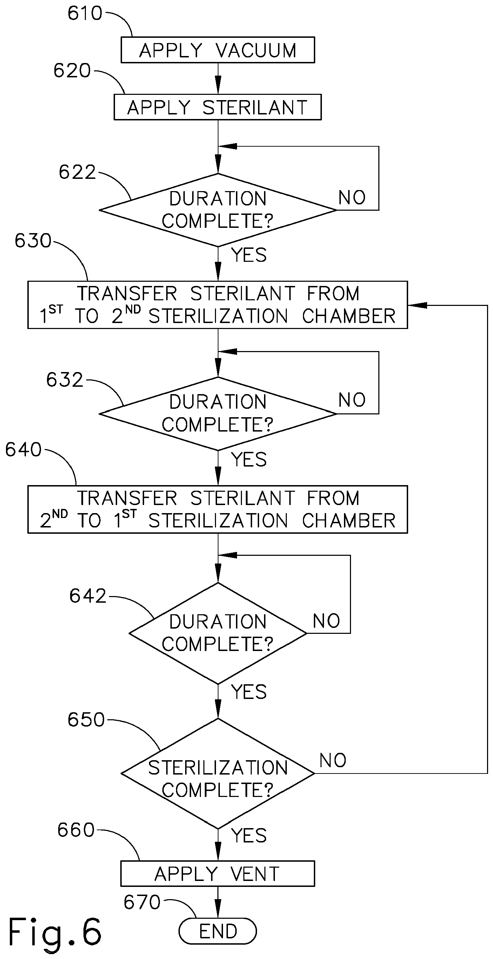

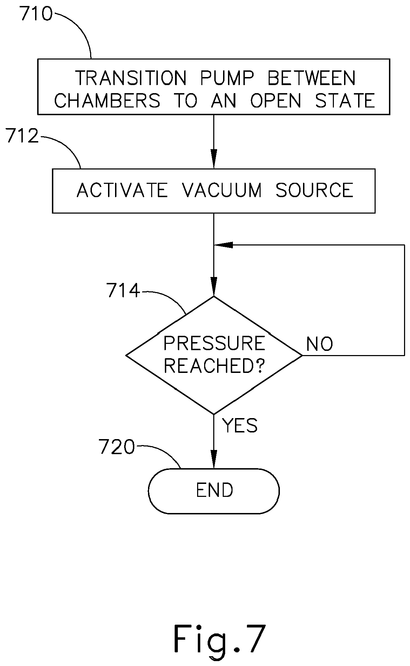

In some versions, sterilization cycle (block 208) includes the exemplary sub-steps shown in FIG. 3. FIGS. 6 and 7 show exemplary alternative sub-steps that may be performed during sterilization cycle (block 208). In the example shown in FIG. 3, the cycle begins with a vacuum being applied (block 310) within sterilization chamber (152). In order to provide such a vacuum, processor (162) may activate vacuum source (180) in accordance with a control algorithm. Processor (162) will then determine (block 312) whether a sufficient vacuum pressure level has been reached within sterilization chamber (152). By way of example only, processor (162) may monitor data from one or more pressure sensors within sterilization chamber (152) as part of determination step (block 312). Alternatively, processor (162) may simply activate vacuum source (180) for a predetermined time period and assume that the appropriate pressure has been reached in sterilization (152) based upon the duration for which vacuum source (180) is activated. Other suitable ways in which processor (162) may determine (block 312) whether a sufficient pressure level has been reached within sterilization chamber (152) will be apparent to those of ordinary skill in the art in view of the teachings herein. Until the appropriate pressure level has been reached within sterilization chamber (152), vacuum source (180) will remain activated.

Once sterilization chamber (152) reaches an appropriate pressure level (e.g., between about 0.2 torr and about 10 torr), processor (162) then activates sterilization module (156) to apply a sterilant (block 314) in sterilization chamber (152). This stage of the process may be referred to as the "transfer phase." By way of example only, the sterilant may comprise a vapor of oxidizing agent such as hydrogen peroxide, peroxy acids (e.g. peracetic acid, performic acid, etc.), ozone, or a mixture thereof. Furthermore, the sterilant may comprise chlorine dioxide. Various other suitable forms that the sterilant may take are described herein; while other forms will be apparent to those of ordinary skill in the art in view of the teachings herein. It should also be understood that, in some versions, the sterilant may be applied (block 314) in different ways based on the user's selection of cycle (block 200) as described above.

Once the sterilant has been applied (block 314) to sterilization chamber (152), processor (162) monitors the time (block 316) to determine whether a sufficient, predetermined duration has passed. By way of example only, this predetermined duration may be anywhere from a few seconds to several minutes. Until the predetermined duration has passed, sterilization chamber (152) remains in a sealed state at the above-noted predetermined pressure level, with the applied sterilant acting upon the medical device(s) contained within sterilization chamber (152). In some variations, processor (162) may monitor data from one or more pressure sensors within sterilization chamber (152) to conform whether a sufficient vacuum pressure is being maintained within sterilization chamber (152).

After the predetermined duration has passed, processor (162) activates (block 318) venting valve (182) to vent sterilization chamber (152) to atmosphere. In some versions, sterilization chamber (152) is allowed to reach atmospheric pressure, while in other versions sterilization chamber (152) only reaches sub-atmospheric pressure. The venting stage of the process may be referred to as the "diffusion phase." In the present example, the sterilization cycle is then complete (block 320) after completion of the diffusion phase. In some other instances, vacuum is again applied to sterilization chamber (152) after completion of the diffusion phase; and then a plasma is applied to sterilization chamber (152), It should be understood that the entire sterilization cycle shown in FIG. 3 (including the above-noted variation where a subsequent vacuum then sterilization are applied) may be repeated one or more times after being completed once. In other words, a medical device may remain within sterilization chamber (152) and experience two or more iterations of the entire cycle shown in FIGS. 3 (including the above-noted variation where a subsequent vacuum then sterilization are applied). The number of iterations may vary based on the cycle selection (block 200), which may be influenced by the particular kind of medical device that is being sterilized in sterilization chamber (152).

Upon completion of the sterilization cycle (block 208), sterilization cabinet (150) may cycle the results (block 210) of the sterilization cycle (block 208). For instance, if sterilization cycle (block 208) was canceled or unable to complete due to error or by a user action, sterilizing cabinet (150) may remain sealed and may also display a sterilization cycle cancellation message via touch screen display (160); as well as various details relating to the sterilization cycle, such as date, time, configuration, elapsed time, sterilization cycle operator, the stage at which the sterilization cycle failed, and other information that may be used to identify why the sterilization cycle. If sterilization cycle (block 208) is completed successfully, sterilization cabinet (150) may display a notification via touch screen display (160) indicating successful completion of sterilization cycle (block 208). In addition, sterilization cabinet (150) may display information such as sterilization cycle identifier, sterilization cycle type, start time, duration, operator, and other information (666).

In some variations, a pre-plasma may be applied in the sterilization cycle (block 208) to heat up the medical device contained in sterilization chamber (152). By way of example only, plasma may be applied between applying a vacuum (block 310) and applying sterilant (block 314). In addition, or in the alternative, a post-plasma may be applied at the end of sterilization cycle (block 208) to degrade any residual sterilant that may be adsorbed to the surface of the medical device contained in sterilization chamber (152). It should be understood that, before applying the post-plasma, a vacuum would first need to be applied to sterilization chamber (152).

By way of example only, the process depicted in FIG. 3 may be carried out at temperatures where the walls of sterilization chamber (152) are between about 30°C and about 56°C, or more particularly between about 47°C and about 56°C, or even more particularly about 50°C; and where the temperature of the medical device in sterilization chamber (152) is between about 5-10°C and about 40-55°C.

In addition to the foregoing, sterilizing cabinet (150) may be configured to perform sterilization processes in accordance with at least some of the teachings of U.S. Pat. No. 6,939,519, the disclosure of which is incorporated by reference herein; U.S. Pat. No. 6,852,279, the disclosure of which is incorporated by reference herein; U.S. Pat. No. 6,852,277, the disclosure of which is incorporated by reference herein; U.S. Pat. No. 6,447,719, the disclosure of which is incorporated by reference herein; U.S. Pat. No. 6,365,102, the disclosure of which is incorporated by reference herein; U.S. Pat. No. 6,325,972, the disclosure of which is incorporated by reference herein; and/or U.S. Provisional Patent App. No. 62/316,722, the disclosure of which is incorporated by reference herein.

While the foregoing examples are described in the context of sterilizing medical devices, and particularly endoscopes, it should be understood that the teachings herein may also be readily applied in the context of sterilizing various other kinds of articles. The teachings are not limited to endoscopes or other medical devices. Other suitable articles that may be sterilized in accordance with the teachings herein will be apparent to those of ordinary skill in the art.

### III. Overview of Exemplary Sterilization System with Independent Vacuum Chambers

As noted above, after sterilant vapor is applied (block 314) in a sterilization chamber (152) and remains in sterilization chamber (152) for a certain period of time, the sterilant is vented to atmosphere (block 318). It should be understood that the vented sterilant vapor is lost to the atmosphere and cannot be re-used. Thus, if another sterilization cycle (block 208) is desired, new sterilant vapor must be added into sterilization chamber (152). Once the additional sterilization cycle (block 208) is completed, the sterilant vapor is once again vented to the atmosphere and cannot be re-used. Sterilization of medical devices having long, narrow lumens may require multiple sterilization cycles (block 208). Consequently, multiple doses of new sterilant vapor may be required in order to provide for sufficient sterilization.

In view of the foregoing, it may be desirable to provide a variation of sterilization cabinet (150) that enables re-use of the same sterilant vapor, thereby enabling repetition of sterilization cycles (block 208) while avoiding (or at least reducing) the loss of sterilant while repeating sterilization cycles (block 208). In addition, or in the alternative, it may be desirable to provide a variation of sterilization cabinet (150) that allows dynamic adjustment of sterilant dosage to account for sterilant depletion that occurs via breakdown, absorption, adsorption, reaction, condensation, etc. A merely illustrative example of a variation of sterilization cabinet (150) that may be used to provide either or both of these functionalities is described in greater detail below.

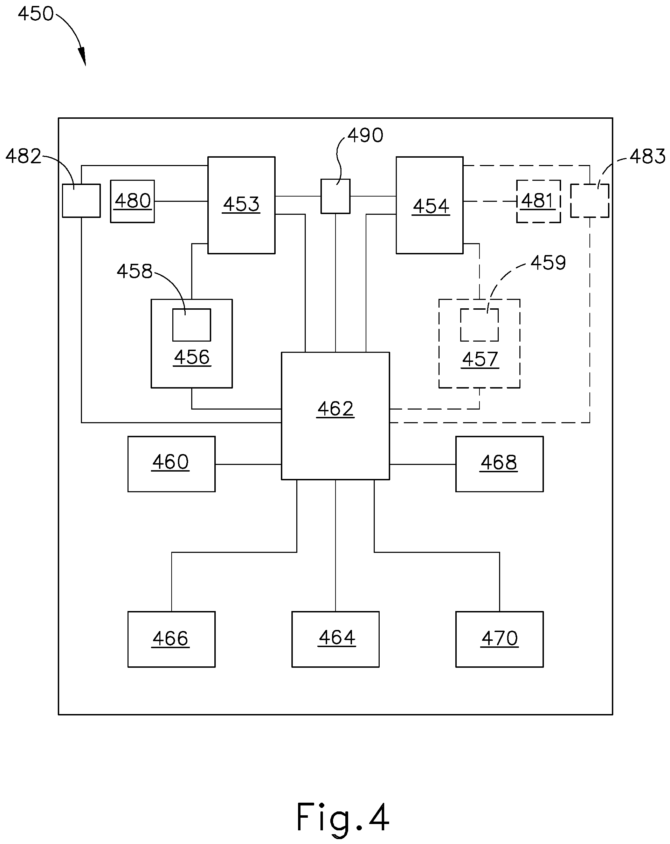

FIG. 4 depicts an exemplary sterilizing cabinet (450) that is operable to sterilize two or more medical devices, such as endoscopes, etc., simultaneously. Sterilizing cabinets may comprise at least two sterilization chambers. Sterilizing cabinet (450) of the present example comprises two sterilization chambers (453, 454), which are each configured to receive at least one medical device for sterilization. While not shown, sterilizing cabinet (450) also comprises two doors. Each door opens and closes a respective one of sterilization chambers (453, 454) in response to actuation of one or more corresponding kick plates (not shown). An operator may thereby open and close sterilization chambers (453, 454) in a hands-free fashion. In some versions, sterilization chamber (453) may be opened and closed independently of sterilization chamber (454), and vice versa. Of course, any other suitable features may be used to provide selective access to sterilization chambers (453,454).

It should also be understood that, in some versions, one sterilization chamber (453) may be nested within the other sterilization chamber (454). As another merely illustrative variation, sterilization chambers (453, 454) may be positioned adjacent to each other, with one door being operable to selectively open and close both sterilization chambers (453, 454) simultaneously. As still another merely illustrative variation, sterilization chambers (453, 454) may be positioned adjacent to each other, with a sliding wall separating sterilization chamber (453) from sterilization chamber (454). In particular, when the sliding wall translates in a first direction, the sliding wall may reduce the capacity of sterilization chamber (453) while simultaneously increasing the capacity of sterilization chamber (454). When the sliding wall translates in a second direction, the sliding wall may reduce the capacity of sterilization chamber (454) while simultaneously increasing the capacity of sterilization chamber (453). The sliding wall may thus be configured like a piston in a double acting cylinder. Moreover, the sliding wall may provide transfer of sterilant from sterilization chamber (453) to sterilization chamber (454), and from sterilization chamber (454) to sterilization chamber (453), as will be described in greater detail below.

Sterilizing cabinets may also comprise a pump in fluid communication with the at least two sterilization chambers. Sterilizing cabinet (450) of the present example comprises pump (490) that is in fluid communication with first sterilization chamber (453) and second sterilization chamber (454). Pump (490) is configured to transfer sterilant vapor from first sterilization chamber (453) to second sterilization chamber (454) and *vice versa.* Various suitable components and configurations that may be used to provide pump (490), including the capability of selectively pumping in a selected one of two opposite directions, will be apparent to those of ordinary skill in the art in view of the teachings herein. It should also be understood that sterilization chambers (453, 454) may be configured such that one or lumens of endoscopes or other medical devices disposed in sterilization chambers (453, 454) provide a pathway for sterilant to pass between sterilization chambers (453, 454) as described below. In other words, sterilization chambers (453,454) may be configured such that sterilant must pass through one or lumens of endoscopes or other medical devices disposed in sterilization chambers (453, 454) in order for the sterilant to pass between sterilization chambers (453, 454) as described below. In addition, or in the alternative, sterilization chambers (453, 454) may be configured such that sterilant must pass through one or lumens of endoscopes or other medical devices disposed in sterilization chambers (453, 454) in order for the sterilant to pass from sterilization modules (456, 457) to sterilization chambers (453, 454) as described below.

In some variations, two separate pumps are used-one pump to transfer sterilant vapor from first sterilization chamber (453) to second sterilization chamber (454) and another pump to transfer sterilant vapor from second sterilization chamber (454) to first sterilization chamber (453). In some versions, sterilization chambers (453, 454) may be selectively vented (by activating venting valves (482, 483) to assist in transfer of vapor from first sterilization chamber (453) to second sterilization chamber (454) and *vice versa.*

Sterilizing cabinets may also comprise at least one sterilization module that is operable to dispense sterilant into one or more of the at least two sterilization chambers in order to sterilize medical devices contained in the sterilization chambers. Sterilizing cabinet (450) of the present example comprises first sterilization module (456) and optional second sterilization module (457). First sterilization module (456) is operable to dispense sterilant into first sterilization chamber (453) and second sterilization module (457) is operable to dispense sterilant into second sterilization chamber (454). In the present example, first sterilization module (456) is configured to receive replaceable sterilant cartridges (458) containing a certain amount of sterilant, and second sterilization module (457) is configured to receive replaceable sterilant cartridges (459) containing a certain amount of sterilant. By way of example only, each sterilant cartridge (458, 459) may each contain enough sterilant to perform one to fifty or five to ten sterilization procedures. Alternatively, the sterilant may be provided in a bulk form (e.g., in a large bottle or other container and dosed into chamber (453, 454) in a specified amount).

In the present example, first sterilization module (456) and second sterilization module (457) are each operable to respectively apply sterilant in the form of vapor within first and second sterilization chambers (453, 454). By way of example only, sterilization modules (456, 457) may comprise a combination of a vaporizer and a condenser. The vaporizer may include a chamber that receives a particular concentration of sterilant solution (e.g., a liquid hydrogen peroxide solution with a concentration of about 20% to about 59% nominal, or between about 20% to 59%, or or between about 58% and about 59.6%); where sterilant solution changes phase from liquid to vapor on re-vaporization. The condenser may provide condensation of sterilant solution vapor, and the concentration of sterilant solution may be thereby increased (e.g., from about 59% nominal to somewhere between about 83% nominal and about 95% nominal), by removal of water vapor. Alternatively, any other suitable methods and components may be used to apply sterilant in the form of a vapor within first and second sterilization chambers (453, 454).

In any case, to supplement the application of sterilant in the form of a vapor, sterilant may also be applied (in liquid form) to the inside of lumen(s) and/or other internal spaces within medical devices and/or outside of medical devices, before medical devices are placed in first and second sterilization chambers (453, 454). In such versions, sterilant may evaporate while a vacuum is applied to first and second sterilization chambers (453, 454) (e.g., as described in greater detail below with reference to block 610 of FIG. 6) and even after vacuum is applied; and provide more sterilant to areas of medical devices with less penetration range, thereby further promoting effective sterilization.

In some variations, only one sterilization module (456, 457) is provided. It should be understood from the description below that a single sterilization module (456, 457) may provide sufficient sterilization for use in both sterilization chambers (453, 454), such that each sterilization chamber (453, 454) does not need a corresponding dedicated sterilization module (456, 457). It should also be understood that two or more sterilization modules (456, 457) may be arranged to dispense sterilant into a single sterilization chamber (453) in an alternating fashion, with sterilization chamber (454) receiving the sterilant from sterilization chamber (453). Thus, sterilization chamber (453) may serve as a primary chamber while sterilization chamber (454) serves as a secondary chamber.

Sterilizing cabinet (450) of the present example further comprises a touch screen display (460). Touch screen display (460) is operable to render the various user interface display screens, such as those described in U.S. Provisional Pat. App. No. 62/316,722, the disclosure of which is incorporated by reference herein. Of course, touch screen display (460) may display various other screens as well. Touch screen display (460) is further configured to receive user input in the form of user contacting touch screen display (460) in accordance with conventional touch screen technology. In addition, or in the alternative, sterilizing cabinet (450) may include various other kinds of user input features, including but not limited to buttons, keypads, keyboards, a mouse, a trackball, etc.

Sterilizing cabinets may further comprise a processor that is in communication with at least one sterilization module and with a touch screen display. In the present example, processor (462) is in communication with first sterilization module (456), with optional second sterilization module (457), and with touch screen display (460).

In accordance with user input, processor (462) is operable to execute control algorithms to drive at least one sterilization module (456), and is optionally operable to execute control algorithms to drive optional second sterilization module(s) (457). Processor (462) is further operable to execute instructions to display the various screens on touch screen display (460); and to process instructions received from a user via touch screen display (460) (and/or via other user input features). Processor (462) is also in communication with various other components of sterilizing cabinet (450) and is thereby operable to drive those components and/or process input and/or other data from those components. Various suitable components and configurations that may be used to form processor (462) will be apparent to those of ordinary skill in the art in view of the teachings herein.

In the example shown in FIG. 4, processor (462) is in communication with pump (490) and processor (462) is operable to selectively activate pump (490) to transfer sterilant vapor from first sterilization chamber (453) to second sterilization chamber (454) and *vice versa.* As described in greater detail below, in such versions, pump (490) may first transfer sterilant vapor from first sterilization chamber (453) to second sterilization chamber (454) and then subsequently transfer the sterilant vapor from second sterilization chamber (454) back to first sterilization chamber (453).

Sterilizing cabinet (450) further comprises an identification tag reader (466), which is operable to read an identification tag of a biological indicator as described herein. By way of example only, identification tag reader (466) may comprise an optical reader that is operable to read an optical identification tag (e.g., barcode, QR code, etc.) of a biological indicator. In addition, or in the alternative, identification tag reader (466) may comprise RFID reader that is operable to read an RFID identification tag (e.g., barcode, QR code, etc.) of a biological indicator. Various suitable components and configurations that may be used to form identification tag reader (466) will be apparent to those of ordinary skill in the art in view of the teachings herein. Data received through identification tag reader (466) is processed through processor (462).

Sterilizing cabinet (450) of the present example further comprises a memory (468), which is operable to store control logic and instructions and that are executed by processor (462) to drive components such as sterilization modules (456, 457), touch screen display (460), communication module (464), and identification tag reader (466). Memory (468) may also be used to store results associated with setup of a sterilization cycle, performance of a load conditioning cycle, performance of a sterilization cycle, and/or various other kinds of information. Various suitable forms that memory (468) may take, as well as various ways in which memory (468) may be used, will be apparent to those of ordinary skill in the art in view of the teachings herein.

Sterilizing cabinet (450) of the present example further comprises a printer (470), which is operable to print information such as results associated with setup of a sterilization cycle, performance of a load conditioning cycle, performance of a sterilization cycle, and/or various other kinds of information. By way of example only, printer (470) may comprise a thermal printer, though of course any other suitable kind of printer may be used. Various suitable forms that printer (470) may take, as well as various ways in which printer (470) may be used, will be apparent to those of ordinary skill in the art in view of the teachings herein. It should also be understood that printer (470) is merely optional and may be omitted in some versions.

Sterilizing cabinet (450) of the present example further comprises at least one vacuum source (480) and at least one venting valve (482). Vacuum source (480) is in fluid communication with at least first sterilization chamber (453) and is also in communication with processor (462). In some variations, a single vacuum source (480) provides vacuum for first and second sterilization chambers (453, 454). However, in the example shown in FIG. 4, first vacuum source (480) is in fluid communication with first sterilization chamber (453) and first venting valve (482); and optional second vacuum source (481) is in fluid communication with second sterilization chamber (454) and second venting valve (483). Thus, each sterilization chamber (453, 454) has its own dedicated vacuum source (480, 481) in the present example, though as noted above, both sterilization chambers (453, 454) may share a single common vacuum source (480) in other variations.

In the present example, processor (462) is operable to selectively activate vacuum source (480) in accordance with one or more control algorithms. Processor (462) is further operable to selectively activate vacuum source (481) in accordance with one or more additional control algorithms. When vacuum source (480) is activated, vacuum source (480) is operable to reduce pressure within sterilization chamber (453) as will be described in greater detail below. In versions, where vacuum source (480) is in communication with both sterilization chambers (453, 454), vacuum source (480) is operable to reduce pressure within both sterilization chambers (453, 454). In versions where each sterilization chamber (453, 454) has its own dedicated vacuum source (480, 481), when second vacuum source (481) is activated, vacuum source (481) is operable to reduce pressure within second sterilization chamber (454) as will be described in greater detail below.

In some versions, only one vacuum source (480, 481) is provided. It should be understood that, since sterilization chambers (453, 454) are in fluid communication with each other via pump (490), a single vacuum source (480, 481) may be used to create a vacuum in both sterilization chambers (453, 454). In some such versions, pump (490) may provide a simple open pathway between sterilization chambers (453, 454) when vacuum source (480, 481) is activated. In some other versions, pump (490) may also be activated simultaneously with the single vacuum source (480, 481) to thereby cooperate with the single vacuum source (480, 481) to provide a vacuum in both sterilization chambers (453, 454). Other suitable arrangements and relationships that may be used for vacuum source (480, 481) and pump (490) will be apparent to those of ordinary skill in the art in view of the teachings herein. In some versions, pump (490) also acts as a vacuum pump for chamber (453) and/or for chamber (454).

At least one venting valve (482) is also in fluid communication with sterilization chamber (453). In addition, at least one venting valve (482) is in communication with processor (462) such that processor (462) is operable to selectively activate venting valve (482) in accordance with one or more control algorithms. In the example shown in FIG. 4, optional second venting valve (483) is also in communication with processor (462) such that processor (462) is operable to selectively activate optional second venting valve (483) in accordance with one or more control algorithms.

When at least one venting valve (482) is activated, venting valve (482) is operable to vent sterilization chamber (453). In the example shown in FIG. 4, when either or both of first venting valve (482) and optional second venting valve (483) are activated, venting valves (482, 483) are respectively operable to vent first and second sterilization chambers (453, 454) to atmosphere as will be described in greater detail below. Various suitable components that may be used to provide vacuum sources (480, 481) and venting valves (482, 483) will be apparent to those of ordinary skill in the art in view of the teachings herein.

In some versions, only one venting valve (482, 483) is used to vent both sterilization chambers (453, 454). It should be understood that since sterilization chambers (453, 454) are in fluid communication with each other via pump (490), pump (490) may be configured to provide an open fluid pathway between the sterilization chambers (453, 454) to enable the venting of both sterilization chambers (453, 454) through single venting valve (482, 483). Other suitable arrangements and relationships that may be used for venting valve (482, 483) and pump (490) will be apparent to those of ordinary skill in the art in view of the teachings herein.

In addition to the foregoing, sterilizing cabinet (450) may be configured and operable in accordance with at least some of the teachings of U.S. Pat. No. 6,939,519, the disclosure of which is incorporated by reference herein; U.S. Pat. No. 6,852,279, the disclosure of which is incorporated by reference herein; U.S. Pat. No. 6,852,277, the disclosure of which is incorporated by reference herein; U.S. Pat. No. 6,447,719, the disclosure of which is incorporated by reference herein; U.S. Pat. No. 6,365,102, the disclosure of which is incorporated by reference herein; U.S. Pat. No. 6,325,972, the disclosure of which is incorporated by reference herein; and/or U.S. Provisional Patent App. No. 62/316,722, the disclosure of which is incorporated by reference herein.

### IV. Overview of Exemplary Sterilization Process Utilizing Independent Vacuum Chambers

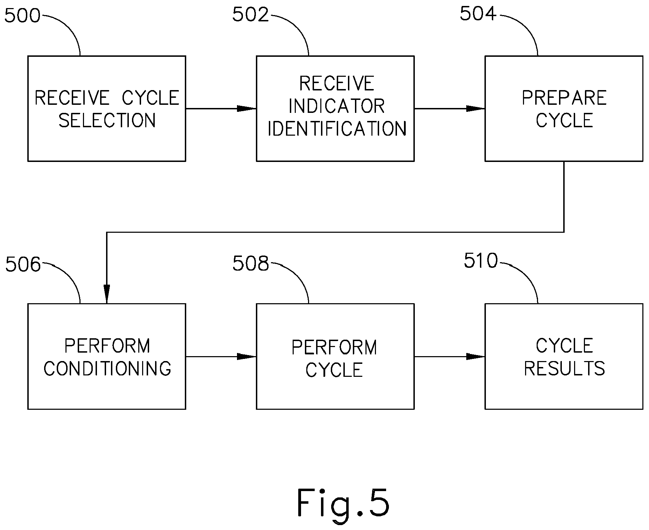

FIG. 5 depicts a high level flowchart of an exemplary set of steps that sterilizing cabinet (450) could perform to sterilize at least two medical devices, for example, two endoscopes. Sterilizing cabinet (450) may be configured to perform one or more sterilization cycles, with different sterilization cycles being appropriate for different types and quantities of medical devices. Thus, as an initial step, sterilizing cabinet (450) may display one or more available sterilization cycles via touch screen display (460) and then receive a sterilization cycle selection (block 500) from user.

Sterilizing cabinet (450) may also display instructions indicating whether a biological indicator should be used with selected sterilization cycle, and receive a biological indicator identification (block 502). Such a biological indicator identification (block 502) may be provided via identification tag reader (466), via touch screen display (460), or otherwise. A biological indicator may be placed inside at least one or all sterilization chambers (453, 454) of sterilizing cabinet (450) before sterilization cycle begins and may remain in sterilization chamber(s) (453, 454) during sterilization cycle. In the present example, a separate biological indicator would be placed in each sterilization chamber (453, 454). A user may identify the particular biological indicators (block 502) before the biological indicators are placed in sterilization chambers (453, 454). As noted above, the biological indicators may contain microorganisms that are responsive to a particular sterilization cycle. Upon completion of sterilization cycle, the biological indicators may be tested for the microorganisms in order to provide a measure of the effectiveness of the sterilization cycle.

Selection of a sterilization cycle (block 500) and identification of a biological indicator (block 502) may define one or more requirements for the configuration and arrangement of medical devices within at least one sterilization chamber (453, 454). Thus, in order to provide preparation for sterilization cycle (508), once sterilization cycle has been selected (block 500) and biological indicator has been identified (block 502), sterilizing cabinet (450) may provide a display via touch screen display (460) indicating a proper medical device placement. This display may serve as a visual guide to a user's placement of medical device(s) (and perhaps a biological indicator) within at least two sterilization chambers (453, 454) of sterilizing cabinet (450), based on sterilization cycle selection (block 500). The doors of sterilization chambers (453, 454) may be opened to enable the user to place medical devices (and perhaps biological indicators) within sterilization chambers (453, 454) as instructed.

Once the user has placed medical devices in chambers (453, 454) based on these instructions, the user may press a start button or other button indicating that medical device placement is complete. In some versions, sterilizing cabinet (450) is configured to automatically verify proper medical device placement. By way of example only, sterilizing cabinet (450) may employ photo sensors, imaging devices, weight sensors, and/or other components to verify proper medical device placement in sterilization chambers (453, 454). It should be understood, however, that some versions of sterilizing cabinet (450) may lack the capability of automatically verifying proper placement of a medical device within sterilization chambers (453, 454).

If medical device placement is verified and/or the user has otherwise completed cycle preparation (block 504), sterilization chambers (453, 454) may start a load conditioning process (block 506). Load conditioning process (block 506) prepares sterilization chambers (453, 454) and medical device(s) within sterilization chambers (453, 454) for optimal sterilization during a sterilization cycle. Conditioning may include controlling and optimizing one or more characteristics of sterilization chambers (453, 454). For example, during load conditioning, sterilizing cabinet (450) may continuously monitor level of moisture within sterilization chambers (453, 454) while reducing level of moisture by, for example, circulating and dehumidifying air of sterilization chambers (453, 454), creating a vacuum within sterilization chambers (453, 454), heating sterilization chambers (453, 454), and/or other methods for dehumidifying a sealed chamber. This may continue until sterilizing cabinet (450) determines that an acceptable level of moisture has been reached.

As part of load conditioning cycle (block 506), sterilizing cabinet (450) may also continuously detect temperature within sterilization chambers (453, 454) while heating sterilization chambers (453, 454) by, for example, convection of heated air, conduction through an interior surface of chambers (453, 454), and/or using other techniques. This may continue until sterilizing cabinet (450) determines that an acceptable internal temperature has been reached. Various conditioning actions in one or more sterilization chambers (453, 454) such as controlling temperature or humidity, may be performed in parallel or in sequence. It should also be understood that load conditioning cycle (block 506) may verify that sterilization chambers (453, 454) are sealed; verifying contents of sterilization chambers (453, 454); checking physical characteristics of contents of sterilization chambers (453, 454) such as content volume, content weight, or other characteristics; and/or performing one or more conditioning steps that may include chemical treatment, plasma treatment, or other types of treatment to reduce moisture, raise temperature, and/or otherwise prepare medical devices in sterilization chambers (453, 454) for sterilization cycle (block 508).

While one or more conditioning actions are being performed as part of load conditioning cycle (block 506), sterilizing cabinet (450) may display information via touch screen display (460) indicating to user the duration of time before sterilization cycle (block 508) performance may begin. Once all load conditioning criteria have been successfully met, load conditioning cycle (block 506) is complete, and sterilization cycle (block 508) may then be performed. It should therefore be understood that sterilizing cabinet (450) is configured such that sterilization cycle (block 508) is not actually initiated until after load conditioning cycle (block 506) is complete. It should also be understood that load conditioning cycle (block 506) may be omitted or varied in some versions of sterilizing cabinet (450) operation.

As noted above, sterilizing cabinet (450) may begin performing sterilization cycle (block 508) automatically and immediately after load conditioning (block 506) has been completed. Sterilization cycle (block 508) may include exposing medical device(s) in sterilization chambers (453, 454) to pressurized sterilant gas, further heat treatment, chemical treatment, plasma treatment, vacuum treatment, and/or other types of sterilization procedures. During performance of sterilization cycle (block 508), sterilizing cabinet (450) may display information via touch screen display (460) such as a duration remaining for sterilization cycle (block 508), the current stage of sterilization cycle (block 508) (e.g. plasma, vacuum, injection, heat, chemical treatment), and/or other information.

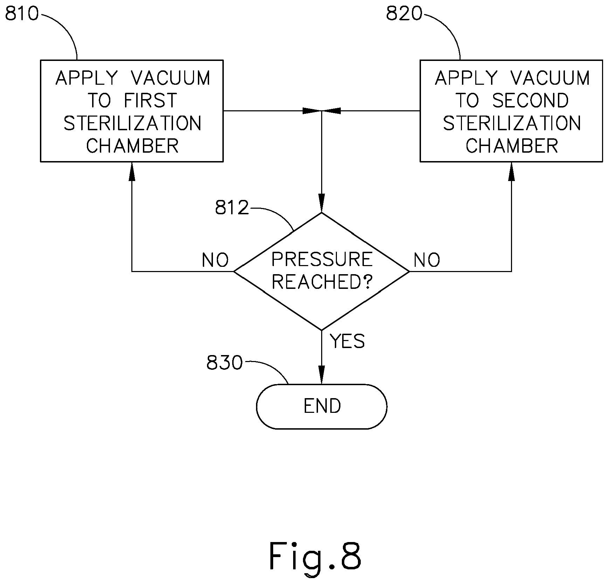

In some versions, sterilization cycle (block 508) comprises the set of exemplary sub-steps shown in FIG. 6. In particular, sterilization cycle (block 508) begins with applying vacuum (block 610) to sterilization chambers (453, 454). FIG. 7 shows one exemplary set of sub-steps that may be used to apply vacuum (610) to sterilization chambers (453, 454) using just a single vacuum source (480). FIG. 8 shows another exemplary set of sub-steps that may be used to apply vacuum (610) to sterilization chambers (453, 454) using two vacuum sources (480, 481). Each of these exemplary methods will be described in greater detail below. However, it should be understood that various other suitable methods may be used to apply vacuum (610) to sterilization chambers (453, 454). Other alternative methods will be apparent to those of ordinary skill in the art in view of the teachings herein.

In versions of sterilizing cabinet (450) having only one vacuum source (480), applying vacuum (block 610) may occur via the sub-steps shown in FIG. 7. In these versions, processor (462) is operable to transition pump (490) between an open state, in which there is an open fluid communication pathway between sterilization chambers (453, 454), and a closed state, in which the fluid communication pathway between sterilization chambers (453, 454) is closed. Various suitable configurations that may be used to provide selection between these two states will be apparent to those of ordinary skill in the art in view of the teachings herein. In some other variations, a separate valve is used to selectively provide an open path for fluid communication between chambers (453, 454), such that pump (490) is not used to selectively provide an open path for fluid communication between chambers (453, 454). Returning to the present example, in accordance with a control algorithm, processor (462) transitions pump (490) to the open state (block 710) and then activates vacuum source (480) (block 712) to provide vacuum to first and second sterilization chambers (453, 454).

Processor (462) will then determine (block 714) whether a sufficient pressure level has been reached within first sterilization chamber (453) and/or second sterilization chamber (454). By way of example only, processor (462) may monitor data from one or more pressure sensors within first and second sterilization chambers (453, 454) as part of determination step (block 714). Alternatively, processor (462) may simply activate vacuum source (480) for a predetermined time period and assume that appropriate pressures have been reached in first and second sterilization chambers (453, 454) based upon the duration for which vacuum source (480) is activated. Other suitable ways in which processor (462) may determine (block 714) whether sufficient pressure levels have been reached within first and second sterilization chambers (453, 454) will be apparent to those of ordinary skill in the art in view of the teachings herein. Until the appropriate pressure levels have been reached within first and second sterilization chambers (453, 454), vacuum source (480) will remain activated, and pump (490) will remain in an open state.

Once first and second sterilization chambers (453, 454) both reach an appropriate pressure level, the vacuum cycle ends (block 720). At this stage, sterilizing cabinet (450) may proceed with applying a sterilant (block 620) as shown in FIG. 6 and as described in greater detail below.

In versions of sterilizing cabinet (450) having only one vacuum source (480), applying vacuum (block 610) may alternatively occur as follows. While vacuum source (480) is activated, pump (490) is in an open state and drives air from second sterilization chamber (454) into first sterilization chamber (453), thereby assisting in creation of a vacuum in second sterilization chamber (454). Processor (462) is operable to monitor the pressure level in one or both chambers (453, 454). Once the appropriate pressure level has been reached in second sterilization chamber (454), processor (462) may deactivate vacuum source (480) and transition pump (490) to a closed state, thereby closing the fluid communication pathway between sterilization chambers (453, 454). Of course, pump (490) and vacuum source (480) may likewise be activated by processor (462) to force air from, and create a vacuum in, first sterilization chamber (453). In any case, at this stage, sterilizing cabinet (450) may proceed with applying a sterilant (block 620) as shown in FIG. 6 and as described in greater detail below.

In versions of sterilizing cabinet (450) comprising at least two vacuum sources (480, 481), applying vacuum (block 610) may occur via the sub-steps shown in FIG. 8. In particular, processor (462) may activate vacuum source (480) in accordance with a control algorithm to apply vacuum (block 810) to first sterilization chamber (453). In parallel or in sequence, processor (462) may activate vacuum source (481) to apply vacuum (block 820) to second sterilization chamber (454). It should be understood that processor (462) may activate vacuum sources (480, 481) in reverse order, i.e., to apply vacuum (block 810) to second sterilization chamber (454) before applying vacuum to first sterilization chamber (453); or activate both vacuum sources (480, 481) simultaneously.

After activating vacuum sources (480, 481) (blocks 810, 820), processor (462) will then determine (block 812) whether a sufficient pressure level has been reached within first sterilization chamber (453) and/or second sterilization chamber (454). By way of example only, processor (462) may monitor data from one or more pressure sensors within first and second sterilization chambers (453, 454) as part of determination step (block 812). Alternatively, processor (462) may simply activate vacuum sources (480, 481) for predetermined time periods and assume that appropriate pressures have been reached in first and second sterilization chambers (453, 454) based upon the duration for which vacuum sources (480, 481) are activated. Other suitable ways in which processor (462) may determine (block 812) whether sufficient pressure levels have been reached within first and second sterilization chambers (453, 454) will be apparent to those of ordinary skill in the art in view of the teachings herein. Until the appropriate pressure levels have been reached within first and second sterilization chambers (453, 454), vacuum sources (480, 481) will remain activated. Once first and second sterilization chambers (453, 454) both reach an appropriate pressure level, vacuum cycle (block 610) ends (block 830). At this stage, sterilizing cabinet (450) may proceed with applying a sterilant (block 620) as shown in FIG. 6 and as described in greater detail below.

In some versions, once first and second sterilization chambers (453, 454) reach an appropriate pressure level (e.g., between about 0.2 torr and about 10 torr), sterilization cycle (block 508) continues by applying sterilant (block 620) to first sterilization chamber (453) while maintaining vacuum in second sterilization chamber (454). Thus, the fluid path between sterilization chambers (453, 454) (via pump (490), via a valve, or otherwise), remains closed. When sterilant is applied (block 620) to first sterilization chamber (453) the pressure inside first sterilization chamber (453) may increase.

By way of example only, sterilant vapor may comprise a vapor of oxidizing agent such as hydrogen peroxide, peroxy acids (e.g. peracetic acid, performic acid, etc.), ozone, or a mixture thereof. Furthermore, sterilant may comprise chlorine dioxide. Various other suitable forms that sterilant may take are described herein; while other forms will be apparent to those of ordinary skill in the art in view of the teachings herein. It should also be understood that, in some versions, sterilant may be applied (block 620) in different ways based on user's selection of cycle (block 500) as described above.

Once sterilant has been applied (block 620) to first sterilization chamber (453), processor (462) monitors time (block 622) to determine whether a sufficient, predetermined duration has passed. By way of example only, this predetermined duration may be anywhere from a few seconds to several minutes. Until the predetermined duration has passed, first and second sterilization chambers (453, 454) remain in a sealed state at the above-noted predetermined pressure level, with applied sterilant acting upon medical device(s) contained within first sterilization chamber (453). Until the predetermined duration has passed, one or more sensors may be used to monitor the pressure inside first sterilization chamber (453) after sterilant is applied (block 620) to first sterilization chamber (453). Moreover, one or more sensors may be used to monitor the pressure inside second sterilization chamber (454) during this same time period.

Once the predetermined duration has passed, the transfer phase begins when processor (462) activates pump (490) to transfer all of the sterilant vapor (block 630) from first sterilization chamber (453) into second sterilization chamber (454). When transferring the sterilant vapor (block 630) from first sterilization chamber (453) to second sterilization chamber (454), the pressure in first sterilization chamber (453) may drop and the pressure in second sterilization chamber (454) may increase. The transfer of sterilant vapor from first sterilization chamber (453) into second sterilization chamber (454) and the associated change in pressure may provide movement and diffusion of the sterilant vapor within a medical device contained in first sterilization chamber (453), thereby providing for increased diffusion of the sterilant vapor into hard-to-reach spaces within the medical device.

Processor (462) monitors time (block 632) to determine whether a sufficient, predetermined duration has passed. By way of example only, this predetermined duration may be anywhere from a few seconds to several minutes. Until the predetermined duration has passed, first and second sterilization chambers (453, 454) remain in a sealed state, with applied sterilant acting upon medical device(s) contained within second sterilization chamber (454). Again, until the predetermined duration has passed, one or more sensors may be used to monitor the pressure inside second sterilization chamber (454) after sterilant is transferred (block 630) to second sterilization chamber (454). Moreover, one or more sensors may be used to monitor the pressure inside first sterilization chamber (453) during this same time period.

Once the predetermined duration has passed, processor (462) activates pump (490) to transfer all or some of sterilant (block 640) from second sterilization chamber (454) to first sterilization chamber (453). When transferring sterilant (block 640) from second sterilization chamber (454) to first sterilization chamber (453), the pressure in second sterilization chamber (454) will drop and the pressure in first sterilization chamber (453) will increase. The transfer of sterilant vapor from second sterilization chamber (454) into first sterilization chamber (453), and vice-versa, and the associated change in pressure will provide movement and diffusion of the sterilant vapor within a medical device in either sterilization chamber (453, 454), thereby providing for increased diffusion of the sterilant vapor into hard-to-reach spaces within the medical device.

Processor (462) monitors time (block 642) to determine whether a sufficient, predetermined duration has passed. By way of example only, this predetermined duration may be anywhere from a few seconds to several minutes. Until predetermined the duration has passed, first and second sterilization chambers (453, 454) remain in a sealed state at the above-noted predetermined pressure level, with applied sterilant acting upon medical device(s) contained within first sterilization chamber (453).

Once the predetermined duration has passed, if sterilization is not complete (block 650), then the process (blocks 630-650) of transferring sterilant (completely or partially) between first and second sterilization chambers (453, 454) may be repeated one or more times after being completed once. In other words, medical device(s) may remain within first and second sterilization chambers (453, 454) and experience two or more iterations of the partial cycle (blocks 630-650). It should also be understood that medical devices may remain within first and second sterilization chambers (453, 454) and experience two or more iterations of the entire cycle (blocks 610-670) shown in FIG. 6. In any case, the number of iterations may vary based on cycle selection (block 200), which may be influenced by the particular kind of medical device(s) that are being sterilized in first and second sterilization chambers (453, 454). It should be noted that during the sterilization cycle (block 508), additional sterilant may need to be added to either one or both of first and second sterilization chambers (453, 454) after one or more instances of transferring (blocks 630, 640) sterilant between chambers (453, 454).

Once sterilization is complete (block 650), the sterilant vapor may be present in either or both of sterilization chambers (453, 454). The diffusion phase begins when processor (462) activates (block 660) venting valves (482, 483) to vent first and second sterilization chambers (453, 454) to atmosphere. The venting process can be instant or stepwise. By way of example only, stepwise venting may be provided where venting valve (482, 483) is opened for a few milliseconds and then closed, and after a few seconds or minutes, the opening and closing is repeated again one or more times, and then sterilization chamber (453, 454) is eventually fully vented to atmosphere. In some versions, first and second sterilization chambers (453, 454) are allowed to reach atmospheric pressure, while in other versions, first and second sterilization chambers (453, 454) only reach sub-atmospheric pressure. In the present example, the sterilization cycle (block 508) is then complete (block 670) after completion of the diffusion phase.

Upon completion of sterilization cycle (block 508), sterilizing cabinet (450) may process the results (block 510) of sterilization cycle (block 508). For instance, if sterilization cycle (block 508) was canceled or unable to complete due to error or by user action, sterilizing cabinet (450) may remain sealed and may also display a sterilization cycle cancellation message via touch screen display (460); as well as various details relating to sterilization cycle (block 508), such as date, time, configuration, elapsed time, sterilization cycle operator, the stage at which sterilization cycle failed, and other information that may be used to identify why sterilization cycle (block 508) was not completed. If sterilization cycle (block 508) is completed successfully, sterilizing cabinet (450) may display a notification via touch screen display (460) indicating successful completion of sterilization cycle (block 608). In addition, sterilizing cabinet (450) may display information such as sterilization cycle identifier, sterilization cycle type, start time, duration, operator, and other information.

In some variations, pre-plasma may be applied in sterilization cycle (block 508) to heat up medical device(s) contained in sterilization chambers (453, 454). By way of example only, plasma may be applied between applying a vacuum (block 610) and applying sterilant (block 620). In addition, or in the alternative, a post-plasma may be applied at the end of sterilization cycle (block 650) to degrade any residual sterilant that may be adsorbed to the surface of any medical devices contained in first and second sterilization chambers (453, 454). It should be understood that, before applying the post-plasma, a vacuum would first need to be applied to first and second sterilization chambers (453,454).

By way of example only, the process depicted in FIG. 6 may be carried out at temperatures where walls of first and second sterilization chambers (453, 454) are between about 30°C and about 60-70°C, or more particularly between about 47°C and about 56°C, or even more particularly about 50°C; and where temperature of medical device(s) in first and second sterilization chambers (453, 454) is between about 5-10°C and about 40-55°C. Moreover, the process depicted in FIG. 6 may be carried out when one or more medical devices are present in only one of sterilization chambers (453, 454). In other words, the process depicted in FIG. 6 is effective regardless of whether medical devices are present in both sterilization chambers (453, 454). It should therefore be understood that the process depicted in FIG. 6 may be carried out with one or more medical devices being positioned only in first sterilization chamber (453) or only in second sterilization chamber (454).

In some variations, only one chamber (453) is configured to receive one or more medical devices while the other chamber (454) is not configured to receive one or more medical devices. In such versions, the other chamber (454) may simply be used to push sterilant vapor into, and to pull sterilant vapor back from, in order to minimize consumption of sterilant during repetition of sterilization cycles (block 208).

It should be understood from the foregoing that, like the venting step (block 318) in the sterilization process (block 208) described above with reference to FIG. 3, the transfer of the sterilant vapor back and forth (blocks 630, 640) between first and second chambers (453, 454) in the process described above with reference to FIG. 6 provides movement of the sterilant vapor within the medical device(s) that is/are contained in chamber(s) (453,454), resulting in diffusion of the sterilant vapor into hard-to-reach spaces (e.g., long, narrow lumens) within such medical device(s). However, unlike the venting step (block 318) in the sterilization process (block 208) described above with reference to FIG. 3, the transfer of the sterilant vapor back and forth (blocks 630, 640) between first and second chambers (453, 454) in the process described above with reference to FIG. 6 results in the re-use of sterilant vapor during iterations of sterilization cycles rather than the release of sterilant vapor into the atmosphere during iterations of sterilization cycles. The re-use of the sterilant vapor advantageously allows for repeated sterilization cycles of a medical device to be undertaken without the addition of subsequent full doses of sterilant into a chamber (453, 454) (though some nominal, lesser amount of sterilant may need to be introduced into chamber (453 or 454) after one or more iterations of the sterilization cycle in order to "top off" chamber (453 or 454) with sterilant). Thus, the process shown in FIG. 6 may provide better conservation of sterilant, or more efficient use of sterilant, which may be particularly beneficial for sterilization of medical devices that warrant repeated sterilization cycles (e.g., endoscopes with long, narrow lumens).

In some variations, sterilant is first dispensed into both sterilization chambers (453, 454), and after a few seconds to a few minutes, pump (490) transfers some or almost all of the sterilant from sterilization chamber (453) to sterilization chamber (454), and after a few seconds to a few minutes, pump (490) transfers some or almost all of the sterilant from sterilization chamber (454) to sterilization chamber (453), and this process is repeated as many times as needed. Once the process has been repeated for the desired number of times, both sterilization chambers (453, 454) are vented by air either instantly or in a stepwise manner.

In addition to the foregoing, sterilizing cabinet (450) may be configured to perform sterilization processes in accordance with at least some of the teachings of U.S. Pat. No. 6,939,519, the disclosure of which is incorporated by reference herein; U.S. Pat. No. 6,852,279, the disclosure of which is incorporated by reference herein; U.S. Pat. No. 6,852,277, the disclosure of which is incorporated by reference herein; U.S. Pat. No. 6,447,719, the disclosure of which is incorporated by reference herein; U.S. Pat. No. 6,365,102, the disclosure of which is incorporated by reference herein; U.S. Pat. No. 6,325,972, the disclosure of which is incorporated by reference herein; and/or U.S. Provisional Patent App. No. 62/316,722, the disclosure of which is incorporated by reference herein.

### V. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A sterilization system comprising: (a) a first chamber comprising a first venting valve, wherein the first chamber is configured to receive a first medical device, wherein the first chamber is further operable to sterilize the first medical device; and (b) a second chamber; (c) a first sterilization module that is operable to dispense sterilant into the first chamber; and (d) a pump assembly in fluid communication with the first chamber and the second chamber, wherein the pump assembly is operable to selectively and repeatedly: (i) transfer at least some of the sterilant from the first chamber to the second chamber, and (ii) transfer at least some of the sterilant from the second chamber to the first chamber.

### Example 2

The sterilization system of Example 1, wherein the second chamber is configured to receive a second medical device and wherein the second chamber is operable to sterilize the second medical device.

### Example 3

The sterilization system of any one or more of Examples 1 through 2, further comprising a first vacuum source in fluid communication with the first chamber, wherein first vacuum source is operable to reduce pressure in the first chamber.

### Example 4

The sterilization system of Example 3, further comprising a processor in communication with the first vacuum source and the first venting valve, wherein the processor is operable to selectively activate the first vacuum source to reduce pressure in the first chamber or to vent the first chamber.

### Example 5

The sterilization system of Example 4, wherein the processor is in further communication with the pump assembly, wherein the processor is operable to selectively activate the pump assembly to transfer the sterilant from the first chamber to the second chamber or to transfer the sterilant from the second chamber to the first chamber.

### Example 6

The sterilization system of any one or more of Examples 1 through 5, wherein the second chamber comprises a second venting valve.

### Example 7

The sterilization system of Example 6, further comprising a second vacuum source in fluid communication with the second chamber, wherein second vacuum source is operable to reduce pressure in the second sterilization chamber.

### Example 8

The sterilization system of Example 7, further comprising a processor in communication with the second vacuum source and the second venting valve, wherein the processor is operable to selectively activate the second vacuum source to reduce pressure in the second chamber or to vent the second chamber.

### Example 9

The sterilization system of any one or more of Examples 1 through 8, wherein the first and second chambers are further operable to condition respective first and second medical devices by detecting moisture on the first and second medical devices and removing moisture from the first and second medical devices.

### Example 10

The sterilization system of any one or more of Examples 1 through 9, wherein the pump assembly is operable to transition to an open state where the pump assembly provides an open pathway between the first chamber and the second chamber to enable simultaneous venting of the first chamber and the second chamber.

### Example 11

The sterilization system of any one or more of Examples 1 through 10, further comprising at least one venting valve, wherein the at least one venting valve is operable to selectively vent the first chamber and the second chamber instantly or in a stepwise manner.

### Example 12

The sterilization system of any one or more of Examples 1 through 11, further comprising a second sterilization module that is operable to dispense sterilant into the second chamber.

### Example 13

The sterilization system of any one or more of Examples 1 through 12, wherein the first chamber and the second chamber are configured to provide fluid communication between the first and second chamber via a fluid passageway formed in the medical device, wherein the pump assembly is operable to transfer at least some of the sterilant from the first chamber to the second chamber and transfer at least some of the sterilant from the second chamber to the first chamber via the fluid passageway formed in the medical device.

### Example 14

The sterilization system of any one or more of Examples 1 through 13, wherein the first chamber is configured to receive one or more endoscopes.

### Example 15

The sterilization system of any one or more of Examples 1 through 14, wherein the pump assembly comprises at least two pumps.

### Example 16

A sterilizing cabinet that is operable to sterilize at least one endoscope, the sterilizing cabinet comprising: (a) a first chamber, wherein the first chamber is configured to receive a first endoscope and is operable to sterilize the first endoscope; (b) a first sterilization module that is operable to dispense sterilant into the first chamber; (c) a second chamber, wherein the second chamber is configured to receive sterilant from the first chamber; (d) a first vacuum source in fluid communication with the first chamber, wherein first vacuum source is operable to reduce pressure in the first chamber; and (f) at least one pump in fluid communication with the first chamber and the second chamber, wherein the at least one pump is operable to selectively provide an open fluid pathway between the first chamber and the second chamber when the first vacuum source is activated, wherein the at least one pump is further operable to selectively transfer at least some of the sterilant from the first chamber to the second chamber, wherein the at least one pump is further operable to selectively transfer at least some of the sterilant from the second chamber to the first chamber.

### Example 17

The sterilizing cabinet of Example 16, wherein the at least one pump comprises a single pump, wherein the single pump is operable to transfer the sterilant from the first chamber to the second chamber, wherein the single pump is further operable to transfer the sterilant from the second chamber to the first chamber.

### Example 18

The sterilizing cabinet of any one or more of Examples 16 through 17, wherein the at least one pump comprises a first pump and a second pump, wherein the first pump is operable to transfer the sterilant from the first chamber to the second chamber, wherein the second pump is further operable to transfer the sterilant from the second chamber to the first chamber.

### Example 19

A method of sterilizing a medical device in a sterilizing cabinet, the sterilizing cabinet comprising a first chamber and a second chamber, the method comprising: (a) placing a medical device in the first chamber; (b) applying vacuum to the first chamber and the second chamber; (c) applying sterilant to the first chamber; (d) transferring the sterilant from the first chamber to the second chamber; (e) transferring the sterilant from the second chamber to the first chamber; and (f) venting the first chamber and the second chamber.

### Example 20

The method of Example 19, wherein applying vacuum to the first chamber and the second chamber comprises: (i) opening a fluid communication pathway between the first chamber and the second chamber, (ii) applying a vacuum to the first chamber and the second chamber simultaneously, and (iii) closing the fluid communication pathway between the first chamber and the second chamber.

### Example 21

The method of one or more of Examples 19 through 20, further comprising applying additional sterilant to the first chamber after transferring sterilant from the first chamber to the second chamber.

### VI. Miscellaneous

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A sterilization system comprising:
(a) a first chamber comprising a first venting valve, wherein the first chamber is configured to receive a first medical device, wherein the first chamber is further operable to sterilize the first medical device; and
(b) a second chamber;
(c) a first sterilization module that is operable to dispense sterilant into the first chamber; and
(d) a pump assembly in fluid communication with the first chamber and the second chamber, wherein the pump assembly is operable to selectively and repeatedly:
(i) transfer at least some of the sterilant from the first chamber to the second chamber, and
(ii) transfer at least some of the sterilant from the second chamber to the first chamber.

2. The sterilization system of claim 1, wherein the second chamber is configured to receive a second medical device and wherein the second chamber is operable to sterilize the second medical device.

3. The sterilization system of claim 1, further comprising a first vacuum source in fluid communication with the first chamber, wherein first vacuum source is operable to reduce pressure in the first chamber.

4. The sterilization system of claim 3, further comprising a processor in communication with the first vacuum source and the first venting valve, wherein the processor is operable to selectively activate the first vacuum source to reduce pressure in the first chamber or to vent the first chamber.

5. The sterilization system of claim 4, wherein the processor is in further communication with the pump assembly, wherein the processor is operable to selectively activate the pump assembly to transfer the sterilant from the first chamber to the second chamber or to transfer the sterilant from the second chamber to the first chamber.

6. The sterilization system of claim 2, wherein the second chamber comprises a second venting valve.

7. The sterilization system of claim 6, further comprising a second vacuum source in fluid communication with the second chamber, wherein second vacuum source is operable to reduce pressure in the second sterilization chamber.

8. The sterilization system of claim 7, further comprising a processor in communication with the second vacuum source and the second venting valve, wherein the processor is operable to selectively activate the second vacuum source to reduce pressure in the second chamber or to vent the second chamber.

9. The sterilization system of claim 2, wherein the first and second chambers are further operable to condition the first and second medical devices by detecting moisture on the first and second medical devices and removing moisture from the first and second medical devices.

10. The sterilization system of claim 1, wherein the pump assembly is operable to transition to an open state where the pump assembly provides an open pathway between the first chamber and the second chamber to enable simultaneous venting of the first chamber and the second chamber.

11. The sterilization system of claim 1, further comprising at least one venting valve, wherein the at least one venting valve is operable to selectively vent the first chamber and the second chamber instantly or in a stepwise manner.

12. The sterilization system of claim 1, further comprising a second sterilization module that is operable to dispense sterilant into the second chamber.

13. The sterilization system of claim 1, wherein the first chamber and the second chamber are configured to provide fluid communication between the first and second chamber via a fluid passageway formed in the medical device, wherein the pump assembly is operable to transfer at least some of the sterilant from the first chamber to the second chamber and transfer at least some of the sterilant from the second chamber to the first chamber via the fluid passageway formed in the medical device.

14. The sterilization system of claim 1, wherein the first chamber is configured to receive one or more endoscopes.

15. The sterilization system of claim 1, wherein the pump assembly comprises at least two pumps.

16. A sterilizing cabinet that is operable to sterilize at least one endoscope, the sterilizing cabinet comprising:
(a) a first chamber, wherein the first chamber is configured to receive a first endoscope and is operable to sterilize the first endoscope;
(b) a first sterilization module that is operable to dispense sterilant into the first chamber
(c) a second chamber, wherein the second chamber is configured to receive sterilant from the first chamber;
(d) a first vacuum source in fluid communication with the first chamber, wherein first vacuum source is operable to reduce pressure in the first chamber; and
(f) at least one pump in fluid communication with the first chamber and the second chamber, wherein the at least one pump is operable to selectively provide an open fluid pathway between the first chamber and the second chamber when the first vacuum source is activated, wherein the at least one pump is further operable to selectively transfer at least some of the sterilant from the first chamber to the second chamber, wherein the at least one pump is further operable to selectively transfer at least some of the sterilant from the second chamber to the first chamber.

17. The sterilizing cabinet of claim 16, wherein the at least one pump comprises a single pump, wherein the single pump is operable to transfer the sterilant from the first chamber to the second chamber, wherein the single pump is further operable to transfer the sterilant from the second chamber to the first chamber.

18. The sterilizing cabinet of claim 16, wherein the at least one pump comprises a first pump and a second pump, wherein the first pump is operable to transfer the sterilant from the first chamber to the second chamber, wherein the second pump is further operable to transfer the sterilant from the second chamber to the first chamber.

19. A method of sterilizing a medical device in a sterilizing cabinet, the sterilizing cabinet comprising a first chamber and a second chamber, the method comprising:
(a) placing a medical device in the first chamber;
(b) applying vacuum to the first chamber and the second chamber;
(c) applying sterilant to the first chamber;
(d) transferring the sterilant from the first chamber to the second chamber;
(e) transferring the sterilant from the second chamber to the first chamber; and
(f) venting the first chamber and the second chamber.

20. The method of claim 19, wherein applying vacuum to the first chamber and the second chamber comprises:
(i) opening a fluid communication pathway between the first chamber and the second chamber,
(ii) applying a vacuum to the first chamber and the second chamber simultaneously, and
(iii) closing the fluid communication pathway between the first chamber and the second chamber.
